(19) **Europäisches Patentamt / European Patent Office / Office européen des brevets**

(11) **EP 3 897 863 B1**

(12) **EUROPEAN PATENT SPECIFICATION**

(45) Date of publication and mention
of the grant of the patent:
**25.01.2023  Bulletin 2023/04**

(51) International Patent Classification (IPC):
*A61K 8/02* (2006.01)     *A61K 8/19* (2006.01)
*A61K 8/46* (2006.01)     *A61K 8/35* (2006.01)
*A61K 8/73* (2006.01)     *A61K 8/49* (2006.01)
*A61K 8/27* (2006.01)     *A61Q 5/12* (2006.01)
*A61Q 5/02* (2006.01)     *A61Q 17/04* (2006.01)
*A61Q 5/00* (2006.01)

(21) Application number: **19806277.0**

(52) Cooperative Patent Classification (CPC):
**A61K 8/19; A61K 8/0241; A61K 8/27; A61K 8/35;
A61K 8/466; A61K 8/4933; A61K 8/736;
A61Q 5/006; A61Q 5/02; A61Q 5/12;**
A61K 2800/58; A61Q 17/04

(22) Date of filing: **26.11.2019**

(86) International application number:
**PCT/EP2019/082627**

(87) International publication number:
**WO 2020/126356 (25.06.2020 Gazette 2020/26)**

(54) **HAIR CARE COMPOSITION COMPRISING PYRITHIONE**

HAARPFLEGEZUSAMMENSETZUNG MIT PYRITHION

COMPOSITION DE SOINS CAPILLAIRES COMPRENANT DE LA PYRITHIONE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018  PCT/CN2018/122794
01.02.2019  EP 19154998**

(43) Date of publication of application:
**27.10.2021  Bulletin 2021/43**

(73) Proprietors:
• **Unilever IP Holdings B.V.
3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **UNILEVER GLOBAL IP LIMITED
Wirral
Merseyside CH62 4ZD (GB)**
Designated Contracting States:
**CY DE GB IE IT MT RS TR**

(72) Inventors:
• **CHEN, Guoqiang
Shanghai, Changning District 200335 (CN)**
• **PAN, Xiaoyun
Shanghai, Changning District 200335 (CN)**
• **TANG, Xuezhi
Shanghai, Changning District 200335 (CN)**

(74) Representative: **Tansley, Sally Elizabeth
Unilever PLC
Unilever Patent Group
Bronland 14
6708 WH Wageningen (NL)**

(56) References cited:
**EP-A2- 1 140 033        WO-A1-2007/065575
WO-A1-2014/124066     WO-A1-2018/033328
WO-A1-2018/172121**

EP 3 897 863 B1

## Description

### Field of the Invention

The present invention relates to hair care compositions. More particularly the invention relates to hair care compositions that comprise pyrithione such as zinc pyrithione.

### Background of the Invention

Zinc pyrithione (ZPT or ZPTO) is an antimicrobial agent which is active against grampositive as well as gram-negative bacteria. It also acts against fungi and yeasts. It is widely used in antimicrobial cleansing compositions and the most common use is in antidandruff (AD) shampoos. Generally, dispersed particles of ZPTO are suspended in the base composition of a shampoo which includes surfactants, water, silicones, polymers and colouring and fragrance ingredients. When a user applies the compositions to hair and scalp, some particles of ZPTO get deposited thereon.

Efficacy of an antidandruff shampoo or conditioner depends on the amount of antidandruff deposited on the scalp and hair because more deposition means more bioavailability. Usually the contact time at the time of application is very short e.g., 10 to 120 seconds before the product gets washed off, so the active does not get much time to deposit. Therefore, it is desirable to maximise its deposition, i.e., deposit as much of the active as possible, in the limited time that is available. However, deposition of actives through a cleansing or wash-off composition presents a technical problem because the particles tend to wash off rather than deposit. An increase in the amount of the active is a possible solution but the solution is neither technically sound nor economically viable. In addition, all the ZPTO that is deposited is not bioavailable as ZPTO tends to react with UV radiation which leads to its destabilization. This destabilization can be avoided to some extent by using UV photostabilisers or sunscreens.

Efforts have also been made to increase the antimicrobial efficacy of ZPT by combining it with "booster" technologies. Certain salts containing zinc have been found to boost the efficacy of ZPTO in shampoo compositions, although their mechanism of action is not fully understood.

However, the inclusion of additional zinc compounds may impair certain properties of the shampoo on dilution, such as flocculation behavior.

US20040213751 A1 (2004, P&G) discloses a composition comprising pyrithione and a zinc-containing layered material which provides an augmentation factor greater than 1.

WO14124066 A1 (P&G) discloses hair care compositions comprising cationic polymers and anionic particulates for improved deposition of pyrithione.

WO03088965 A1 (P&G) discloses a method for delivering excess zinc to eukaryotic cells to inhibit the metabolism of the cell, the method comprising treating the cells with a zinc ionophoric material that is capable of delivering a zinc ion across a cellular membrane wherein the zinc ionophoric material is in combination with a zinc containing material and further wherein there is an increase in an intracellular zinc level by 1.5 fold more than would occur in the absence of the zinc ionophoric material.

US5227156 B1 (Amway Corp) discloses antidandruff shampoo containing thiazolinone preservative and pyrithione. The activity of the preservative is maintained by adding a stabilizer comprising a zinc compound.

WO2018197118 (Unilever) discloses a hair care composition comprising 0.01 to 3% by weight zinc pyrithione; 1 to 5% by weight amino acid; and 0.1 to 5% by weight additional zinc compound. The specific combination of amino acid and selective zinc compound inhibits photo-oxidation and dissociation of ZPTO and further enhances its stabilization.

WO2018172121 (Unilever) discloses a hair care composition comprising 0.01 to 3% by weight zinc pyrithione; an organic UV filter; and 0.1 to 5% by weight additional zinc compound; wherein the weight ratio of zinc compound to zinc pyrithione is over 3:1.

### Summary of the Invention

The present inventors have determined a new way of improving the problem of photo stability of a photolabile antidandruff agent such ZPTO. We have determined that photo stability of ZPTO can be improved if ZPTO, an organic UV filter and a cationic polymer having weight average molecular weight of 1000 Da to 10000000 Da are co-formulated into a composite, which is particulate in nature. The particles may be powdery or granular or even exist in the form of an aqueous dispersion. While hitherto, cationic polymers such as guar gum and chitosan have been associated only with delivery and deposition of active ingredients, we have determined that in the composite in accordance with the invention, the polymer not only ensures delivery and deposition of the photolabile antidandruff active but also improves photo stability of the photolabile active. ZPTO is prone to degradation upon sustained exposure to UV radiation. When ZPTO degrades, it is no longer as effective.

The hypothesis has been verified with ZPTO; a zinc-based photolabile antidandruff agent, but the present inventors believe that the scope of the invention extends to all those photolabile antidandruff agents which are particulate, i.e., exist in the form of particles.

In accordance with a first aspect, disclosed is composite particles comprising a photolabile particulate antidandruff agent and an organic UV filter whose melting point is from 30°C to 100°C, characterized in that said composite particles comprise a cationic polymer having weight average molecular weight of 1000 Da to 10000000 Da.

Preferably the photolabile antidandruff agent is a zinc-based particulate antidandruff agent, especially zinc pyrithione.

In accordance with a second aspect, disclosed is a method of preparing composite particles as claimed in claim 1 comprising a step of heating and agitating an aqueous slurry comprising said organic UV filter and said photolabile antidandruff agent, followed by a step of adding said cationic polymer to said slurry and further heating said slurry for 5 to 60 minutes at 30 to 100°C.

In accordance with another aspect, disclosed is a hair care composition comprising composite particles as claimed in the first aspect.

## Brief Description of the Figures

Fig.1 is Raman spectrum of the composite according to the invention in a haircare (shampoo) composition immediately after preparation.

Fig 2 is Raman spectrum of the composite according to the invention in a haircare (shampoo) composition after storage.

## Detailed Description of the Figures

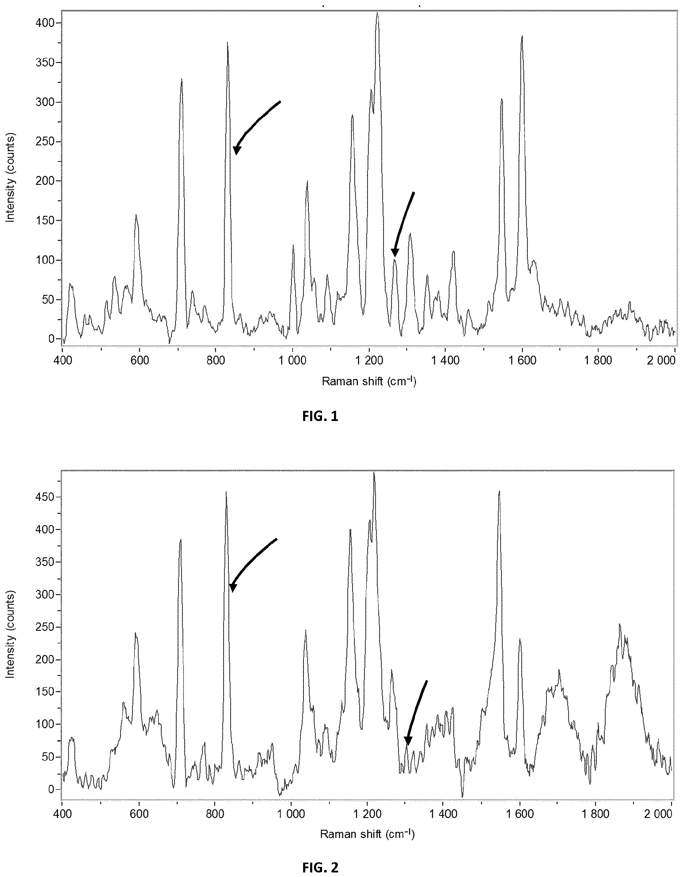

Fig.1 is Raman spectrum of the composite according to the invention in a haircare (shampoo) composition immediately after preparation. The peak at 828 cm$^{-1}$ is characteristic of ZPTO and the one at 1307 cm$^{-1}$ is the peak characteristic of Neo Heliopan® BB, the UV filter.

Fig 2 is Raman spectrum of the composite according to the invention in a haircare (shampoo) composition after storage.

A comparison of the two figures (spectra) indicates that the characteristic peaks are intact from which it can be inferred that not only the composite but the ZPTO also remains intact without any substantial evidence of degradation.

## Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis*. Consequently, features specified in one section may be combined with features specified in other sections as appropriate. Any section headings are added for convenience only, and are not intended to limit the disclosure in any way.

By "hair care composition" as used herein, is meant to include a composition for topical application to hair or scalp of mammals, especially humans. By topical is meant that the composition is applied to the external surface of the body. In the present invention this is achieved by applying the composition on the hair or scalp. Such a composition may be generally classified as leave-on or rinse off, and includes any product applied for improving the appearance, cleansing, odor control or general aesthetics of scalp and hair. The haircare composition of the present invention could be in the form of a liquid, lotion, cream, foam, scrub, gel, shampoo, conditioner, shower gel or bar. The haircare composition of the present invention is preferably a leave-on composition. Alternatively, the haircare composition of the present invention is a wash-off composition. Compositions for achieving the desired benefits by way of ingestion into the human body are excluded from the scope of the present invention.

The composite particles

The composite particles of the invention comprise a photolabile antidandruff agent and an organic UV filter whose melting point is from 30°C to 100°C, characterized in that said composite particles comprise a cationic polymer having weight average molecular weight of 1000 Da to 10000000 Da.

Photolabile means the agent is susceptible of undergoing photochemical reactions under the influence of radiant energy and especially of UV light, including photodegradation, discoloration and the like. It is unstable in the presence of light as opposed to being photostable.

More preferably the photolabile antidandruff agent is a particulate is zinc-based antidandruff agent. More preferably the zinc based antidandruff agent is zinc pyrithione.

Several antidandruff hair care products contain zinc based antidandruff agents like zinc pyrithione (ZPTO, which is a particulate agent). It is found that dissolved intact ZPTO molecules are the sole bioactive form of ZPTO. When hair care product with a zinc based anti-dandruff agent like ZPTO is utilized, the ZPTO dissociates and undergoes photo-oxidation due to which stability of the bioactive is affected due to formation of salts of zinc which reduce the antidandruff efficacy.

Preferably the zinc-based particulate antidandruff agent is zinc pyrithione.

The zinc pyrithione may have any particle form suitable for use in a composition for hair care. For example, the zinc pyrithione may be in the form of amorphous or crystalline particles having a range of particle sizes. The zinc pyrithione may, for example, be in the form of particles having a size distribution in which at least about 90% of the particles have a size of up to 100 microns, more preferably up to 50 microns, even more preferably up to 10 microns, most preferably 5 microns or less.

Amount of the zinc-based antidandruff agent in the composition of the invention would depend on the type of the haircare composition and the precise nature of the antidandruff used. It is preferred that the particles comprise 70 to 90 wt% of said photolabile antidandruff agent, 0.2 to 10 wt% of said organic UV filter and 2 to 10 wt% of said cationic polymer.

The composites of the invention also comprise an organic UV filter whose melting point is from 30°C to 105°C. Preferably the melting point is at least 50°C, more preferably from 50°C to 100°C.

Melting point refers to the temperature at which the solid and liquid forms of a pure substance can exist in equilibrium.

It is preferred that the organic UV filter is oil-soluble. It is further preferred that the organic UV filter is not water-soluble.

Preferably the organic UV filter is at least one of 2-hydroxy-4-methoxybenzophenone (also named as Benzophenone-3 CAS: 131-57-7, MP 62 to 64°C), 2,2-dihydroxy-4-methoxybenzophenone (CAS: 131-53-3, MP 73 to 75°C), butylmethoxydibenzoylmethane (CAS: 70356-09-1, MP 81 to 84°C), bis-ethylhexyloxyphenol methoxyphenyl triazine (Tinosorb S, CAS: 187393-00-6, MP 83-85°C), Menthyl anthranilate (CAS: 134-09-8, MP 62.5-63.5°C), 4-Methylbenzylidene camphor (Enzacamene) (CAS: 36861-47-9, MP 66 to 69°C), Benzophenone-7 (5-chloro-2-hydroxybenzophenone) (CAS: 85-19-8, MP 96 to 98°C), Benzophenone-8 (dioxybenzone) (CAS: 131-53-3, MP 68°C), Benzophenone-10 (mexenone, 2-hydroxy-4-methoxy-4'-methyl-benzophenone, CAS: 1641-17-4, MP 99 to 102°C), Benzophenone-12 (octabenzone) (CAS: 1843-05-6, MP 47 to 49°C).

Further preferably the organic UV filter is a broad-spectrum sunscreen which absorbs UVA as well as UVB radiation.

It is preferred that the amount of the organic UV filter is from 0.01 to 3 wt%, more preferably from about 0.01 to 1.5 wt% by weight of the composite, furthermore preferably from 0.05 to 1.5 wt% by weight of the total composition. In other words, every 100 g of the composite preferably includes from 0.01 to 3 wt% of the organic UV filter, as so on. It is preferred that the ratio of the average amount of the zinc-based antidandruff agent to the average amount of the organic UV filter in the particles is from 1:0.01 to 1:1000 parts by weight. It is further preferred that ratio of average amount of said antidandruff agent to average amount of said organic polymer in said particles is from 1 :0.01 to 1:1000 parts by weight.

The term cationic polymer is used to distinguish such polymers from anionic, i.e., negatively charged polymers as well as from non-ionic polymers, i.e., polymers devoid of any charge.

Zeta potential is the charge that develops at the interface between a solid surface and its liquid medium. This potential, which is measured in MilliVolts, may arise by any of several mechanisms. Among these are the dissociation of ionogenic groups in the particle surface and the differential adsorption of solution ions into the surface region. The net charge at the particle surface affects the ion distribution in the nearby region, increasing the concentration of counterions close to the surface. Thus, an electrical double layer is formed in the region of the particle-liquid interface. Zeta potential is therefore a function of the surface charge of the particle, any adsorbed layer at the interface, and the nature and composition of the surrounding suspension medium. It can be experimentally determined and, because it reflects the effective charge on the particles and is therefore related to the electrostatic repulsion between them, the zeta potential has proven to be extremely relevant to the practical study and control of colloidal stability and flocculation processes. A variety of methods and apparatus are available for its measurement with a reasonable degree of precision. For example, the zeta potential of the particles is measured using a Malvern Nano ZS90 apparatus, in DI water at a solid content of 50 ppm and pH of 7 at 25°C.

It is preferred that zeta potential of the cationic polymer is from +10 to +100 mV. It is further preferred that the polymer is one or more of polyamine, polyvinylpyrrolidone, polylysine, protamine, trimethylammonioethyl (meth)acrylate homopolymers and copolymers, acrylamidopropyl trimethylammonium halide homopolymers and copolymers, dialkyldiallylammonium halide homopolymers and copolymers, chitosan or derivatized chitosan, cellulose or its derivatives comprising trimethyl ammonium substituted epoxide, starch hydroxypropyl trimethyl ammonium halide, polyethyleneimines or polycondensates containing diquaternary ammonium or polyquaternary ammonium repeating units. It is particularly preferred that the polymer is chitosan. The chitosan salt suitable for the present invention comprises a chitosan component and an anion. Preferably the anion is an organic anion and more preferably an organic anion having a molecular weight of greater than 60, more preferably from 80 to 2000, even more preferably from 80 to 500. Preferably, the chitosan salt is a chitosan-amino acid salt. Preferably the amino acid comprises glutamine, glutamic acid, histidine, leucine, lysine, serine, threonine, arginine or a mixture thereof, more preferably comprises arginine. Most preferably, the chitosan salt is chitosan-arginine salt.

More preferably the molecular weight of the cationic polymer is in the range of from 30,000 to 1,000,000 Daltons, even more preferably from 70,000 to 600,000 Daltons, and still even more preferably from 150,000 to 400,000 Daltons. Preferably, the deacetylation degree of chitosan is at least 65%, more preferably from 70 to 95%, even more preferably from 72 to 90% and most preferably from 75 to 85%.

It is particularly preferred that in the composite particles of the invention comprise a photolabile antidandruff agent which is zinc pyrithione, an organic UV filter whose melting point is from 30°C to 105°C which is 2-hydroxy-4-methoxybenzophenone, characterized in that the composite particles comprise a cationic polymer having weight average molecular weight of 1000 Da to 10000000 Da, where the cationic polymer is chitosan.

Preferably, the chitosan comprises at least 5%, more preferably at least 10% of protonated primary amino group by mole of the total amount of primary amino group and protonated primary amino group.

It is preferred that particle size of said particles is from 0.1 $\mu$m to 1000 $\mu$m. A benefit of small particles is that they difficult to spot in clear, transparent formulations. The size may be measured for example, by laser diffraction using a system (such as a Mastersizer™ 2000 available from Malvern Instruments Ltd).

The composite particles of the invention is in the form of an aqueous slurry which may contain from 2 to 70 wt% particles and the balance being water. Alternatively, the composite particles of the invention is in the form of a powder or dry particles which may be obtained by drying or centrifuging the slurry described above.

Method of preparing the composite particles

In accordance with another aspect of the present invention is disclosed a method of preparing composite particles of the first aspect comprising a step of heating and agitating an aqueous slurry comprising said organic UV filter and said photolabile antidandruff agent, followed by a step of adding said cationic polymer to said slurry and further heating said slurry for 5 to 60 minutes at 30 to 100 °C.

Hair care composition

In accordance with a further aspect of the invention is disclosed a hair care composition comprising composite particles of the first aspect. Preferably the composition is a shampoo, hair conditioner, hair cream, hair gel or hair oil. Preferably the amount of the composite particles in such compositions is from 0.5 to 15 wt% of the composition.

Antidandruff agents are compounds that are active against dandruff and are typically antimicrobial agents, preferably antifungal agents. Antidandruff agents typically display a minimum inhibitory concentration of about 50 mg/ml or less against *Malassezia.*

It is preferred that the haircare compositions in accordance with the invention comprises 0.0.5 to 5 wt% of the antidandruff agent. Accordingly such compositions would comprise an appropriately calculated amount of the composite particles.

In addition to the antidandruff agent that is present in the form of the composite particles, the haircare compositions in accordance with this invention may also comprise free additional antidandruff agent which may, for example, be the same as the one contained inside the composite particles. Whenever present, the haircare compositions of the invention preferably comprise 0.0.5 to 5 wt% of the additional antidandruff agent.

The additional antidandruff agent is preferably selected from azoles, Octopirox® (piroctone olamine), selenium sulfide, salicylic acid and combinations thereof. Azoles include ketoconazole and climbazole, preferably climbazole.

The haircare compositions of the invention may further comprise a zinc salt. The additional zinc salt may suitably be selected from zinc salts of organic acids, zinc salts of inorganic acids, zinc oxides, zinc hydroxides or a mixture thereof.

Examples of preferred zinc salts include zinc oxide, zinc pyrrolidone carboxylic acid, zinc citrate, zinc carbonate, zinc chloride, zinc sulphate, zinc glycinate, zinc acetate, zinc lactate, and mixtures thereof. When present, it is preferred that the haircare compositions of the invention comprise 0.1 to 5 wt%, preferably 0.2 to 3 wt%, more preferably from 0.25 to 2.5 wt% of the salt based on the total weight of the composition.

The haircare compositions of the present invention comprises a surfactant selected from the group consisting of anionic surfactants, nonionic surfactants, zwitterionic surfactants and mixtures thereof. The nature, type, amount and specific combinations that may be used depend on the formulation of the composition and would largely depend on whether it is a shampoo or a conditioner or a conditioning shampoo.

Preferably, the haircare compositions of the invention in the form of a shampoo comprise a surfactant which is sodium lauryl sulphate, sodium lauryl ether sulphate, sodium lauryl ether sulphosuccinate, ammonium lauryl sulphate, ammonium lauryl ether sulphate, sodium cocoyl isethionate and lauryl ether carboxylic acid, coco betaine, cocamidopropyl betaine, sodium cocoamphoacetate or a mixture thereof.

Preferably, the haircare composition of the present invention comprises from 1 to 50%, preferably from 2 to 40%, more preferably from 4 to 25% total surfactant.

It is further preferred that the haircare compositions of the invention comprises a cosmetic ingredient. Preferably the cosmetic ingredient is selected from the group consisting of a silicone, an antibacterial agent other than antidandruff agents, a foam booster, a perfume, encapsulates (for example encapsulated fragrance) a dye, a colouring agent, a pigment, a preservative, a thickener, a protein, a phosphate ester, a buffering agent, a pH adjusting agent, a pearlescer (for example; mica, titanium dioxide, titanium dioxide coated mica, ethylene glycol distearate (INCI glycol distearate)) and/or opacifier, a viscosity modifier, an emollient, a sunscreen, an emulsifier, a sensate active (for example menthol and menthol derivatives), vitamins, mineral oils, essential oils, lipids, natural actives, glycerin, natural hair nutrients such as botanical extracts, fruit extracts, sugar derivatives and amino acids, microcrystalline cellulose and mixtures thereof.

Preferably, the haircare composition of the present invention includes from 0.01 to 20 wt% of the at least one cosmetic ingredient, more preferably from 0.05 to 10 wt%, still more preferably from 0.075 to 7.5 wt% and most preferably, from 0.1 to 5 wt% of the at least one cosmetic ingredient, by weight of the total composition.

The haircare composition of the present invention may also comprise synergistic antimicrobial compounds that give synergistic antimicrobial benefit when used in combination with the antidandruff active (for example zinc pyrithione) to enhance its properties and further inhibit the growth of Malassezia furfur. Non-limiting examples of these compounds include compounds having alcoholic groups (e.g. honokiol, magnolol or paeonol), Piperazines and a phenolic compound found in natural plant extract viz. thymol and terpeniol.

The composition may additionally comprise a vitamin B3 compound. The preferred vitamin B3 compound is niacinamide.

Niacinamide is known for secretion of AMPs (Anti-Microbial Proteins) from keratinocytes. The AMPs thus secreted provides for improving immunity of e.g. the scalp. Thus with the use of niacinamide, the anti-dandruff efficacy can be enhanced not just through anti-fungal activity but by boosting the scalp's own protection shield against germs, through use of niacinamide. This combination could provide further long-lasting protection e.g. up to 24 hours of protection against germs.

When present, it is preferred that the haircare compositions of the invention comprise 0.1 to 5% niacinamide, more preferably 0.5 to 5%, furthermore preferably 0.5 to 3%, and optimally 1.0 to 3.0% by weight of the composition.

Silicone

It is preferred that the haircare composition of the invention comprises silicone.

For example, the compositions of the invention may contain emulsified droplets of a silicone conditioning agent for enhancing conditioning performance.

Suitable silicones include polydiorganosiloxanes, polydimethylsiloxanes which have the CTFA designation dimethicone. In addition, suitable for use in the compositions of the invention (particularly shampoos and conditioners) are polydimethyl siloxanes having hydroxyl end groups, which have the CTFA designation dimethiconol.

Preferably the viscosity of the emulsified silicone is at least 10,000 cst at 25 °C, the viscosity of the silicone is preferably at least 60,000 cst, most preferably at least 500,000 cst, ideally at least 1,000,000 cst. Preferably the viscosity does not exceed $10^9$ cst for ease of formulation.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation. A further preferred class of silicones for inclusion in shampoos and conditioners are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

It is preferred that the total amount of silicone is 0.01 to 10 %wt, more preferably 0.1 to 5 wt% and most preferably 0.5 to 3 wt%.

pH of Compositions

It is preferred that pH of the hair care composition of the present invention is preferably from 3 to 7, more preferably 4 to 7, even more preferably 4 to 6.5, most preferably from 4.2 to 6.5.

Shampoos

When the haircare composition of the invention is a shampoo, it is generally aqueous, i.e. they have water or an aqueous solution or a lyotropic liquid crystalline phase as their major component.

Suitably, the shampoo composition comprises 50 to 98%, preferably from 60 to 92% water.

Preferably the shampoo composition comprises one or more cationic polymers for conditioning the hair.

Suitable cationic polymers inclide homopolymers which are cationically substituted or may be formed from two or more types of monomers. The weight average ($M_w$) molecular weight of the polymers will generally be between 100000 and 3 Million Daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine-and/or quaternary ammonium-substituted monomer and/or compatible spacer monomers.

Suitable (non-limiting examples of) cationic polymers include:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides (as described in WO95/22311).

Other cationic polymers that can be used include cationic polysaccharide polymers, such as cationic cellulose derivatives, cationic starch derivatives, and cationic guar gum derivatives.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

It is preferred that the haircare composition of the invention comprises 0.01 to 5%, preferably from 0.02 to 1%, more preferably from 0.05 to 0.8% cationic polymer.

The haircare compositions of the invention may additionally comprise a cationic deposition polymer which is a cationic polygalactomannans having an average molecular weight ($M_w$) of from 1 million to 2.2 million g/mol and a cationic degree of substitution of from 0.13 to 0.3.

The polygalactomannans are polysaccharides composed principally of galactose and mannose units and are usually found in the endosperm material of seeds from leguminous plants such as guar, locust bean, honey locust, flame tree, and other members of the *Leguminosae* family. Polygalactomannans are composed of a backbone of 1→4-linked β-D-mannopyranosyl main chain units (also termed mannoside units or residues) with recurring 1→6-linked α-D-galactosyl side groups (also termed galactoside units or residues) branching from the number 6 carbon atom of a mannopyranose residue in the polymer backbone. The polygalactomannans of the different *Leguminosae* species differ from one another in the frequency of the occurrence of the galactoside side units branching from the polymannoside backbone. The mannoside and galactoside units are generically referred to herein as glycoside units or residues. The average ratio of mannoside to galactoside units in the polygalactomannan contained in guar gum (hereinafter termed "guar") is approximately 2:1.

Suitable cationic polygalactomannans include guar and hydroxyalkyl guar (for example hydroxyethyl guar or hydroxypropyl guar), that has been cationically modified by chemical reaction with one or more derivatizing agents.

In a typical composition the amount of cationic polygalactomannans will generally range from about 0.05 to 1%, preferably from 0.1 to 0.8%, more preferably 0.2 to 0.6% by weight of the composition.

The haircare compositions of the invention may additionally comprise an anionic polymeric rheology modifier such as a carboxylic acid polymer.

The term "carboxylic acid polymer" in the context of this invention generally denotes a homopolymer or copolymer obtained from the polymerization of ethylenically unsaturated monomers containing pendant carboxylic acid groups (hereinafter termed "carboxylic monomers").

Suitable carboxylic monomers generally have one or two carboxylic acid groups, one carbon to carbon double bond and contain a total of from 3 to about 10 carbon atoms, more preferably from 3 to about 5 carbon atoms.

Specific examples of suitable carboxylic monomers include α-β-unsaturated monocarboxylic acids such as acrylic acid, methacrylic acid and crotonic acid; and α-β-unsaturated dicarboxylic acids such as itaconic acid, fumaric acid, maleic acid and aconitic acid. Salts, esters or anhydrides of the α-β-unsaturated mono- or dicarboxylic acids described above may also be used. Examples include half esters of α-β-unsaturated dicarboxylic acids with $C_{1-4}$ alkanols, such as monomethyl fumarate; cyclic anhydrides of α-β-unsaturated dicarboxylic acids such as maleic anhydride, itaconic anhydride and citraconic anhydride; and esters of acrylic acid or methacrylic acid with $C_{1-30}$ alkanols, such as ethyl acrylate, butyl acrylate, 2-ethylhexyl acrylate, dodecyl acrylate, hexadecyl acrylate, and octadecyl acrylate.

Optionally, other ethylenically unsaturated monomers can be copolymerized into the carboxylic acid polymer backbone. Example of such other ethylenically unsaturated monomers include styrene, vinyl acetate, ethylene, butadiene, acrylonitrile and mixtures thereof. Carboxylic acid polymers may preferably have a molecular weight of at least 1 million Daltons.

Suitable examples include crosslinked copolymers polymerized from $C_{1-4}$ alkyl acrylate or methacrylate (e.g. ethyl acrylate) with one or more comonomers selected from acrylic acid, methacrylic acid and mixtures thereof. Such materials may generally be referred to under the INCI name of Acrylates Copolymer. Commercially available examples include Aculyn® 33 from Rohm and Haas.

Also suitable are crosslinked copolymers polymerized from $C_{10-30}$ alkyl esters of acrylic or methacrylic acid with one or more comonomers selected from acrylic acid, methacrylic acid and their respective $C_{1-4}$ alkyl esters. Such materials may generally be referred to under the INCI name of Acrylates/C10-30 Alkyl Acrylate Crosspolymer. Commercially available examples include Carbopol® polymers 1342 and 1382 from Lubrizol Advanced Materials.

Also suitable are optionally crosslinked copolymers of acrylic acid or methacrylic acid with alkyl acrylates and ethoxylated hydrophobically modified alkyl acrylates. Such materials may generally be referred to under the INCI names of Acrylates/Steareth-20 Methacrylate Copolymer, Acrylates/Beheneth-25 Methacrylate Copolymer, Acrylates/Steareth-20 Methacrylate Crosspolymer and Acrylates/Palmeth-25 Acrylates Copolymer. Commercially available examples include Aculyn® 22, 28 or 88 from Rohm & Haas and Synthalen® from 3V Sigma.

It is preferred that the carboxylic acid is a Carbomer, such as homopolymers of acrylic acid crosslinked with an allyl ether of pentaerythritol or an allyl ether of sucrose.

Mixtures of any of the aforementioned materials may also be used.

Preferably the haircare composition of the invention comprises 0.1 to 3.0%, more preferably 0.4 to 1.5% carboxylic acid polymer by weight of the composition.

In formulations containing anionic polymeric rheology modifiers such as the carboxylic acid polymers described above, it is often necessary to neutralize at least a portion of the free carboxyl groups by the addition of an inorganic or organic base. Examples of suitable inorganic or organic bases include alkali metal hydroxides (e.g. sodium or potassium hydroxide), sodium carbonate, ammonium hydroxide, methylamine, diethylamine, trimethylamine, monoethanolamine, triethanolamine and mixtures thereof.

The haircare composition of the invention may also comprise a nonionic polymeric rheology modifier which is selected from one or more nonionic cellulose ethers.

Suitable nonionic cellulose ethers or use as the nonionic polymeric rheology modifier in the invention include ($C_{1-3}$ alkyl) cellulose ethers, such as methyl cellulose and ethyl cellulose; hydroxy ($C_{1-3}$ alkyl) cellulose ethers, such as hydroxyethyl cellulose and hydroxypropyl cellulose; mixed hydroxy ($C_{1-3}$ alkyl) cellulose ethers, such as hydroxyethyl hydroxypropyl cellulose; and ($C_{1-3}$ alkyl) hydroxy ($C_{1-3}$ alkyl) cellulose ethers, such as hydroxyethyl methylcellulose and hydroxypropyl methylcellulose.

Preferred nonionic cellulose ethers for use as the nonionic polymeric rheology modifier in the invention are water-soluble nonionic cellulose ethers such as methylcellulose and hydroxypropyl methylcellulose. The term "water-soluble" in this context denotes a solubility in water of at least 1 grams, more preferably at least 3 grams, most preferably at least 5 grams in 100 grams of distilled water at 25° C. and 1 atmosphere. This level indicates production of a macroscopically isotropic or transparent, coloured or colourless solution.

Methyl cellulose and hydroxypropyl methylcellulose are commercially available in a number of viscosity grades from Dow Chemical as their METHOCEL® trademark series.

Mixtures of any nonionic cellulose ethers may also be suitable. In a typical composition according to the invention the level of nonionic cellulose ethers will generally range from about 0.01 to about 2.0%, and preferably ranges from 0.1 to 0.5%, more preferably from 0.1 to 0.3%, by weight based on the total weight of the composition.

Preferably the haircare composition of the invention comprises 0.1 to 0.3% by weight nonionic cellulose ether.

The haircare composition of the invention may contain further optional ingredients to enhance performance and/or consumer acceptability. Examples of such ingredients include fragrance, dyes and pigments and preservatives. Each of these ingredients will be present in an amount effective to accomplish its purpose. Generally, these optional ingredients are included individually at a level of up to 5% by weight based on the total weight of the composition.

Mode of Use

The haircare composition of the invention is primarily intended for topical application to hair and scalp.

When the composition is a shampoo, it is topically applied to the hair and then massaged into the hair and scalp. Then it is rinsed with water prior to drying the hair. A hair oil or hair serum, being leave-on haircare compositions, are left on for 1 to 10 hours after application before being washed off.

The invention will be further illustrated by the following, non-limiting Examples, in which all percentages quoted are by weight based on total weight unless otherwise stated.

The invention is not limited to the embodiments illustrated in the drawings. Accordingly, it should be understood that where features mentioned in the claims are followed by reference numerals, such numerals are included solely for the purpose of enhancing the intelligibility of the claims and are in no way limiting to the scope of the claims. The examples are intended to illustrate the invention and are not intended to limit the invention to those examples *per se.*

## Examples

Example 1

Preparation of a composite outside the invention (Ref.1)

Ten grams of ZPTO (as 50% aqueous slurry, Ex. Kolon Life Sciences) were charged to a three-necked flask placed inside a water bath. The flask was equipped with mechanical agitator and condenser. The slurry was heated to 85 °C. Thereafter 0.5 g Neo Heliopan® BB (abbreviated as NHBB) was added to the flask. The mixture was heated further and stirred for twenty minutes. Then 89.5 grams deionized water were charged to the flask. After another 20 minutes of continued stirring, the contents were cooled gradually to room temperature. The precipitate was removed. Thereafter a slurry of the composite according to the invention was obtained. The composite was in the form of an aqueous slurry.

The composition of this composite (on 100 g basis) was as follows:

| | |
|---|---|
| ZPTO | = 98.0 wt% |
| Organic UV filter, Neo Heliopan® BB | = 2.0 wt% |

The particle size was found to be 0.6 microns.

Preparation of a composite inside the invention (Ref.2)

Ten grams of ZPTO (as 50% aqueous slurry, Ex. Kolon Life Sciences) were charged to a three-necked flask placed inside a water bath. The flask was equipped with mechanical agitator and condenser. The slurry was heated to 85°C. Thereafter 0.5 g Neo Heliopan® BB (Melting point 65.5°C) was added to the flask. The mixture was heated further and stirred for twenty more minutes. Then 25 grams of a 1 wt% solution of Chitosan acetate and 64.5 grams deionized water were charged to the flask. After another 20 minutes of continued stirring, the contents were cooled gradually to room temperature. The precipitate was removed. Thereafter particles of the composite according to the invention were obtained. The composite was in the form of an aqueous slurry.

The composition of this composite (on 100 g basis solids content) was as follows:

| ZPTO | = 93.4 wt% |
|---|---|
| Organic UV filter, Neo Heliopan® BB | = 1.9 wt% |
| Chitosan 190,000 to 310,000 Da | = 4.7 wt%. |

The particle size was measured and found to be 4 μm.

The particles were thereafter subjected to a variety of tests which are described hereinafter.

Example 2

The compositions/ingredients shown in Table1 below were subjected to an experiment that gives an estimate of the residual amount of ZPTO in the hair care composition after being exposed to UV light for a long time. The %average residual ZPTO was measured using an invitro model which is described hereinafter.

**Table 1**

| Example Ref. No. | ZPTO wt% | NHBB wt% | Zinc acetate wt% | Composite Ref. 1/wt% | Composite Ref. 2/wt% | Model Sebum wt% |
|---|---|---|---|---|---|---|
| C | 0.03 | 0 | 0 | 0 | 0 | To 100 |
| D | 0.03 | 0.0006 | 0 | 0 | 0 | To 100 |
| E | 0.03 | 0.0006 | 0.03 | 0 | 0 | To 100 |
| F | 0 | 0 | 0 | 0.0306 | 0 | To 100 |
| G | 0 | 0 | 0 | 0 | 0.0321 | To 100 |

The composition of the model sebum referred to in the description of the test procedure is shown in Table 2.

**Table 2**

| Lipid | Amount/wt% |
|---|---|
| Oleic acid | 8.0 |
| Isostearic acid | 4.0 |
| Tricaprin | 1.8 |
| Triolein (65%) | 28.2 |
| Triisostearin | 9.2 |
| Oleyl oleate | 11.9 |
| Myristyl myristate | 11.9 |
| Isostearyl isostearate | 6.0 |
| Squalene | 13.8 |
| Cholesterol oleate | 3.4 |
| Cholesterol | 1.7 |

Test to determine UV stability of the composite on Vitro-Skin@

The test conditions are described hereinbelow.

The UV irradiation was carried out in an X-Rite (Macbeth) Spectra Light III chamber. UV mode was chosen for UV irradiation which provides both UVA and UVB light. The UV intensity was fixed by the machine (estimated at 250 $\mu$w/cm$^2$ for UVA and 110 $\mu$w/cm$^2$ for UVB). The transmittance of UVA and UVB in glass vial was 80.3% and 71.9%, respectively. The chamber temperature was equal to the room temperature (20 $\pm$ 2°C). The samples were placed in a line close to the center of the chamber.

Procedure

Example C: Thirty $\mu$L of ZPTO (as 50% aqueous slurry, Ex. Kolon Life Sciences) was dispersed in 200 g deionized water. About 0.5 mL of the so diluted slurry was applied to a plate of Vitro-Skin@ and stirred with a Teflon stirring rod for 30 seconds. The plate was dried naturally under dark conditions Then the plate of treated Vitro-Skin@ was applied with 120 $\mu$L model sebum.

Example D: One mL of ZPTO (as 50% aqueous slurry, Ex. Kolon Life Sciences) was dispersed in 9 g deionized water to form diluted ZPTO slurry. And 1.5 mg Neo Heliopan® BB was dissolved in 0.1 g methanol, and then dispersed into 9.9 g deionized water to form NHBB solution. 30 $\mu$L above diluted ZPTO slurry and 0.2 g above NHBB solution were dispersed into 19.8 g deionized water. About 0.5 mL of the prepared slurry containing ZPTO and NHBB was applied to a plate of Vitro-Skin@ and stirred with a Teflon stirring rod for 30 seconds. The plate was dried naturally under dark condtions. Then the plate of treated Vitro-Skin@ was applied with 120$\mu$L model sebum.

Example E: Two mL of ZPTO (as 50% aqueous slurry, Ex. Kolon Life Sciences) and 1.2 g Zinc acetate dihydrate was dispersed in 16.8 g deionized water to form diluted ZPTO slurry. And 1.5 mg Neo Heliopan® BB was dissolved in 0.1 g methanol, and then dispersed into 9.9 g deionized water to form NHBB solution. 30 $\mu$L above diluted ZPTO slurry and 0.2 g above NHBB solution were dispersed into 19.8 g deionized water. About 0.5 mL of the prepared slurry containing ZPTO, Zinc acetate and NHBB was applied to a plate of Vitro-Skin@ and stirred with a Teflon stirring rod for 30 seconds. The plate was dried naturally under dark conditions. Then the plate of treated Vitro-Skin® was applied with 120$\mu$L model sebum.

Example F: Thirty $\mu$L of a slurry of the composite of Ref.1 containing approximately 5% ZPTO (5 wt% of the slurry) was dispersed in 20 g deionized water. About 0.5 mL of the so diluted slurry was applied to a plate of Vitro-Skin@ and stirred with a Teflon stirring rod for 30 seconds. The plate was dried naturally under dark conditions. Then the plate of treated Vitro-Skin@ was applied with 120 $\mu$L model sebum.

Example G: Thirty $\mu$L of a slurry of the composite of Ref.2 containing approximately 5% ZPTO (5 wt% of the slurry) was dispersed in 20 g deionized water. About 0.5 mL of the so diluted slurry was applied to a plate of Vitro-Skin@ and stirred with a Teflon stirring rod for 30 seconds. The plate was dried naturally under dark conditions. Then the plate of treated Vitro-Skin@ was applied with 120 $\mu$L model sebum.

For each sample, a total of four plates were treated. Two plates of treated Vitro-Skin@ were placed under UV cabinet (Macbeth SpectraLight III) at 37°C for 120 minutes of exposure. The other two plates were kept without UV treatment.

Thereafter, all the four plates were cut off from the holder and immersed in 15 mL centrifuge tubes with 7 mL methanol/water (1:1) each for 10 minutes extraction with ultrasonic treatment.

Then 1 mL of the extracted solution from each sample was taken and treated with 50 $\mu$L of saturated solution of disodium EDTA and 100 $\mu$L of 50 mmol/L 2, 2'-dipyridyl disulfide (DPS) solution for 30 minutes derivatization in dark. The mixture was then passed through a 0.2 $\mu$m PTFE filter and transferred to a Liquid Chromatography sample vial for analysis of ZPTO by UPLC-UV analysis.

A Waters ACQUITYUPLC System coupled to a Quattro Micro API mass spectrometer (Waters, Manchester, UK) was used for the sample analysis. Separation was carried out on a Waters Acquity UPLC BEH C18 column (2.1 mm x 50 mm x 1.7 $\mu$m). The mobile phase was composed of 20 mM ammonium acetate in water and methanol programmed in the linear gradient mode. Atmospheric pressure chemical ionization (APCI) in positive mode was used for all experiments. The multiple reaction monitoring (MRM) mode was used for the determination of ZPTO.

Using the information/observations of the experiments, the stability of ZPTO was determined by the following equation.

$$\text{Stability of ZPTO} \; = \; \frac{\text{Amount of ZPTO after exposure to UV radiation for 2 hours}}{\text{Amount of ZPTO without exposure to UV radiation}}$$

The observations are summarised in Table 3.

**Table 3**

| Example Ref. No. | Details | Stability of ZPTO |
|---|---|---|
| Example C | ZPTO | Control |
| Example D | ZPTO+NHBB | No change over control |
| Example E | ZPTO + NHBB + Zinc acetate | No change over control |
| Example F | Composite Ref.1 | 10% increase over control |
| Example G | Composite of Ref.2 | 40% increase over control |

The data contained in Table 3 clearly indicates that stability of ZPTO improves markedly when the composite in accordance with this invention is used (Example G - Composite of Ref.2) during the experiments. On the other hand, the composite of Ref.1 (which is outside this invention) also improves the stability of ZPTO but the extent of improvement over control is not good enough. The data further indicates that while cationic polymers such as chitosan and chitosan acetate have been reported for use to enhance the deposition of antidandruff agents such as ZPTO, their role in improving UV-stability of photolabile antidandruff agents such as ZPTO has now been demonstrated. Comparison between Example F and Example G further indicates and underlines the importance and surprising effect of chitosan to stabilize ZPTO as the Composite of Ref.1 which contained the sunscreen but not chitsosan failed to provide a decent amount of UV stabilisation.

Example 3

Stability of the Composite Ref. 2 in a haircare composition (shampoo)

The formulations that were tested are shown in Table 4.

**Table 4**

| Ingredient | Comparative Composition | Inventive Composition |
|---|---|---|
| SLES.1EO | 12.00 | 12.00 |
| CAPB | 1.6 | 1.6 |
| ZPTO (50 wt% solids slurry from Kolon) | 10.00 | Absent |
| Composite Ref.2 | Absent | 10.70 |
| NaCl | 1.00 | 1.00 |
| Water and other minors to | To 100 | To 100 |

Test procedure

First, the procedure was followed with the comparative composition. Then it was followed with the inventive composition to find out the difference(s) in performance.

Ten grams of the test composition described in Table 4 was added into 50 mL centrifuge tube, and put into a dark oven at 40°C. After two weeks of storage, 10 g deionized water was added to the tube and mixed evenly. Particles in the composition were collected by centrifugation at 10000 rpm for 20 minutes.

The collected particles were doped on an Al pan and covered by coverslip. A 100x oil objective was used to visualize the ZPTO composite particles. When the particles (approx. 5 to 10 $\mu$m in diameter) were in focus, Raman imaging was conducted by using a 532 nm laser source. The imaging was conducted in a point-by-point manner with a step size of 1 $\mu$m. In each step (pixel), Raman spectrum was obtained by averaging four spectra, with a total of four seconds laser exposure time. The set of spectra was baseline-corrected and analyzed by a built-in multi-variate function to demonstrate distribution of ZPTO and NHBB in the composite. Raman spectra of ZPTO and NHBB were used as standards for comparison.

As far as the inventive composition was concerned, the observations indicated that the composite Ref.2 was stable in the composition even after being stored for two weeks at 40°C. The observations are presented in the form of Raman Spectral data of the composite Ref.2 in the test composition immediately after preparation of the composition (Fig.1) and after storage (Fig.2). The peak at Raman Shift of 1307 cm $^{-1}$ is the peak characteristic of Neo Heliopan® BB and peak at 828 cm$^{-1}$ is characteristic of ZPTO. In each spectrum the peaks have been indicated by arrows.

**Claims**

1. Composite particles comprising a photolabile antidandruff agent and an organic UV filter whose melting point is from 30°C to 105°C, **characterized in that** said composite particles comprise a cationic polymer having weight average molecular weight of 1000 Da to 10000000 Da.

2. Composite particles as claimed in claim 1 wherein said photolabile antidandruff agent is particulate in nature.

3. Composite particles as claimed in claim 1 or 2 wherein said particles comprise 70 to 90 wt% photolabile antidandruff agent, 0.2 to 5 wt% organic UV filter and 1 to 10wt% cationic polymer.

4. Composite particles as claimed in any of claims 1 to 3 wherein ratio of average amount of said antidandruff agent to the average amount of said organic UV filter in said particles is from 1:0.01 to 1:1000 parts by weight.

5. Composite particles as claimed in any of claims 1 to 4 wherein ratio of average amount of said antidandruff agent to average amount of said organic polymer in said particles is from 1:0.01 to 1:1000 parts by weight.

6. Composite particles as claimed in any of claims 1 to 5 wherein said antidandruff agent is zinc pyrithione.

7. Composite particles as claimed in any of claims 1 to 6 wherein said melting point is at least 50°C.

8. Composite particles as claimed in any of claims 1 to 7 wherein said organic UV filter is at least one of 2-hydroxy-4-methoxybenzophenone, 2,2-dihydroxy-4-methoxybenzophenone, butylmethoxydibenzoylmethane, bis-ethylhexyloxyphenol methoxyphenyl triazine, Menthyl anthranilate, 4-Methylbenzylidene camphor, Benzophenone-7, Benzophenone-8, Benzophenone-10 or Benzophenone-12.

9. Composite particles as claimed in claim 7 wherein said organic UV filter is a broad-spectrum sunscreen which absorbs UVA as well as UVB radiation.

10. Composite particles as claimed in any of claims 1 to 9 wherein zeta potential of said cationic polymer is from +10 to +100 mV.

11. Composite particles as claimed in claim 10 wherein said polymer is one or more of polyamine, polyvinylpyrrolidone, polylysine, protamine, trimethylammonioethyl (meth)acrylate homopolymers and copolymers, acrylamidopropyl trimethylammonium halide homopolymers and copolymers, dialkyldiallylammonium halide homopolymers and copolymers, chitosan or derivatized chitosan, cellulose or its derivatives comprising trimethyl ammonium substituted epoxide, starch hydroxypropyl trimethyl ammonium halide, polyethyleneimines or polycondensates containing diquaternary ammonium or polyquaternary ammonium repeating units.

12. A method of preparing composite particles as claimed in claim 1 comprising a step of heating and agitating an aqueous slurry comprising said organic UV filter and said photolabile antidandruff agent, followed by a step of adding said cationic polymer to said slurry and further heating said slurry for 5 to 60 minutes at 30 to 100 °C.

13. A hair care composition comprising composite particles as claimed in any of claims 1 to 12.

14. A haircare composition as claimed in claim 13 wherein amount of said composite particles is 0.5 to 15 wt%.

15. A hair care composition as claimed in claim 14 wherein said composition is a shampoo, hair conditioner, hair cream, hair gel or hair oil.

**Patentansprüche**

1. Verbundteilchen, umfassend ein fotolabiles Antischuppenmittel und einen organischen UV-Filter, dessen Schmelzpunkt 30°C bis 105°C beträgt, **dadurch gekennzeichnet, dass** die Verbundteilchen ein kationisches Polymer mit einem gewichtsgemittelten Molekulargewicht von 1000 Da bis 10000000 Da umfassen.

2. Verbundteilchen nach Anspruch 1, wobei das fotolabile Antischuppenmittel von Natur aus teilchenförmig ist.

3. Verbundpartikel, wie im Anspruch 1 oder 2 beansprucht, wobei die Teilchen 70 bis 90 Gew.-% fotolabiles Antischuppenmittel, 0,2 bis 5 Gew.-% organischen UV-Filter und 1 bis 10 Gew.-% kationisches Polymer umfassen.

4. Verbundteilchen, wie in irgendeinem der Ansprüche 1 bis 3 beansprucht, wobei das Verhältnis der durchschnittlichen Menge des Antischuppenmittels zu der durchschnittlichen Menge des organischen UV-Filters in den Teilchen 1:0,01 bis 1:1000 Gewichtsteile beträgt.

5. Verbundteilchen, wie in irgendeinem der Ansprüche 1 bis 4 beansprucht, wobei das Verhältnis der durchschnittlichen Menge des Antischuppenmittels zur durchschnittlichen Menge des organischen Polymers in den Teilchen 1:0,01 bis 1:1000 Gewichtsteile beträgt.

6. Verbundteilchen, wie in irgendeinem der Ansprüche 1 bis 5 beansprucht, wobei das Antischuppenmittel Zinkpyrithion ist.

7. Verbundteilchen, wie in irgendeinem der Ansprüche 1 bis 6 beansprucht, wobei der Schmelzpunkt mindestens 50°C beträgt.

8. Verbundteilchen, wie in irgendeinem der Ansprüche 1 bis 7 beansprucht, wobei der organische UV-Filter mindestens eines von 2-Hydroxy-4-methoxybenzophenon, 2,2-Dihydroxy-4-methoxybenzophenon, Butylmethoxydibenzoylmethan, Bis-ethylhexyloxyphenolmethoxyphenyltriazin, Menthylanthranilat, 4-Mythylbenzylidencampher, Benzophenon-7, Benzophenon-8, Benzophenon-10 oder Benzophenon-12 ist.

9. Verbundteilchen, wie im Anspruch 7 beansprucht, wobei der organische UV-Filter ein Breitspektrum-Sonnenschutz ist, der UVA- sowie UVB-Strahlung absorbiert.

10. Verbundteilchen, wie in irgendeinem der Ansprüche 1 bis 9 beansprucht, wobei das Zeta-Potential des kationischen Polymers +10 bis +100 mV beträgt.

11. Verbundteilchen, wie im Anspruch 10 beansprucht, wobei das Polymer darstellt eines oder mehrere von Polyamin, Polyvinylpyrrolidon, Polylysin, Protamin, Trimethylammonioethyl(meth)acrylat-Homopolymeren und -Copolymeren, Acrylamidopropyltrimethylammoniumhalogenid-Homopolymeren und -Copolymeren, Dialkyldiallylammoniumhalogenid-Homopolymeren und -Copolymeren, Chitosan oder derivatisiertem Chitosan, Cellulose oder dessen Derivaten, umfassend Trimethylammonium-substituiertes Epoxyd, Stärkehydroxypropyltrimethylammoniumhalogenid, Polyethyleniminen oder Polykondensaten, enthaltend diquaternäre Ammonium- oder polyquaternäre Ammonium-Wiederholungseinheiten.

12. Verfahren zur Herstellung von Verbundteilchen, wie im Anspruch 1 beansprucht, umfassend einen Schritt des Erhitzens und Rührens einer wässrigen Aufschlämmung, umfassend organischen UV-Filter und das fotolabile Antischuppenmittel, gefolgt von einem Schritt des Zugebens von kationischem Polymer zu der Aufschlämmung und ferner Erhitzen der Aufschlämmung für 5 bis 60 Minuten bei 30 bis 100 °C.

13. Haarpflegezusammensetzung, umfassend Verbundteilchen, wie in irgendeinem der Ansprüche 1 bis 12 beansprucht.

14. Haarpflegezusammensetzung, wie im Anspruch 13 beansprucht, wobei die Menge der Verbundteilchen 0,5 bis 15 Gew.-% beträgt.

15. Haarpflegezusammensetzung, wie im Anspruch 14 beansprucht, wobei die Zusammensetzung ein Shampoo, einen Haarkonditionierer, eine Haarcreme, ein Haargel oder ein Haaröl ist.

**Revendications**

1. Particules composites comprenant un agent antipelliculaire photolabile et un filtre UV organique dont le point de fusion est de 30°C à 105°C, **caractérisées en ce que** lesdites particules composites comprennent un polymère cationique ayant une masse moléculaire moyenne en masse de 1 000 Da à 10 000 000 Da.

2. Particules composites selon la revendication 1, dans laquelle ledit agent antipelliculaire photolabile est de nature particulaire.

3. Particules composites selon la revendication 1 ou 2, dans lesquelles lesdites particules comprennent de 70 à 90 % en masse d'agent antipelliculaire photolabile, 0,2 à 5 % en masse de filtre UV organique et 1 à 10 % en masse de polymère cationique.

4. Particules composites selon l'une quelconque des revendications 1 à 3, dans lesquelles le rapport de quantité moyenne dudit agent antipelliculaire à la quantité moyenne dudit filtre UV organique dans lesdites particules est de 1:0,01 à 1:1 000 parties en masse.

5. Particules composites selon l'une quelconque des revendications 1 à 4, dans lesquelles le rapport de quantité moyenne dudit agent antipelliculaire à quantité moyenne dudit polymère organique dans lesdites particules est de 1:0,01 à 1:1 000 parties en masse.

6. Particules composites selon l'une quelconque des revendications 1 à 5, dans lesquelles ledit agent antipelliculaire est la pyrithione de zinc.

7. Particules composites selon l'une quelconque des revendications 1 à 6, dans lesquelles ledit point de fusion est d'au moins 50°C.

8. Particules composites selon l'une quelconque des revendications 1 à 7, dans lesquelles ledit filtre UV organique est au moins un de : 2-hydroxy-4-méthoxybenzophénone, 2,2-dihydroxy-4-méthoxy-benzophénone, butylméthoxydibenzoylméthane, bis-éthylhexyloxyphénol méthoxyphényltriazine, anthranilate de menthyle, 4-méthylbenzylidène camphre, benzophénone-7, benzophénone-8, benzophénone-10 ou benzophénone-12.

9. Particules composites selon la revendication 7, dans lesquelles ledit filtre UV organique est un écran solaire à spectre large qui adsorbe un rayonnement UVA ainsi qu'UVB.

10. Particules composites selon l'une quelconque des revendications 1 à 9, dans lesquelles le potentiel zêta dudit polymère cationique est de +10 à +100 mV.

11. Particules composites selon la revendication 10, dans lesquelles ledit polymère est un ou plusieurs d'homopolymères et copolymères de polyamine, polyvinylpyrrolidone, polylysine, protamine, (méth)acrylate de triméthylammonioéthyle, homopolymères et copolymères d'halogénure d'acrylamidopropyltriméthylammonium, homopolymères et copolymères d'halogénure de dialkyldiallylammonium, chitosane ou chitosane dérivé, cellulose ou ses dérivés comprenant un époxyde substitué par du triméthylammonium, halogénure d'hydroxypropyltriméthylammonium d'amidon, polyéthylène-imines ou polycondensats contenant des unités répétitives d'ammonium diquaternaire ou d'ammonium polyquaternaire.

12. Procédé de préparation de particules composites selon la revendication 1 comprenant une étape de chauffage et d'agitation d'une suspension aqueuse comprenant ledit filtre UV organique et ledit agent antipelliculaire photolabile, suivie par une étape d'addition dudit polymère cationique à ladite suspension et de chauffage supplémentaire de ladite suspension pendant de 5 à 60 minutes à de 30 à 100°C.

13. Composition pour le soin des cheveux comprenant des particules composites selon l'une quelconque des revendications 1 à 12.

14. Composition pour le soin des cheveux selon la revendication 13, dans laquelle une quantité desdites particules composites est de 0,5 à 15 % en masse.

15. Composition pour le soin des cheveux selon la revendication 14, dans laquelle ladite composition est un shampooing, un après-shampooing, une crème capillaire, un gel capillaire ou une huile capillaire.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040213751 A1
- WO 14124066 A1
- WO 03088965 A1
- US 5227156 B1
- WO 2018197118 A
- WO 2018172121 A
- US 4009256 A
- WO 9522311 A


**Non-patent literature cited in the description**

- *CHEMICAL ABSTRACTS,* 131-57-7
- *CHEMICAL ABSTRACTS,* 131-53-3
- *CHEMICAL ABSTRACTS,* 70356-09-1
- *CHEMICAL ABSTRACTS,* 187393-00-6
- *CHEMICAL ABSTRACTS,* 134-09-8
- *CHEMICAL ABSTRACTS,* 36861-47-9
- *CHEMICAL ABSTRACTS,* 85-19-8
- *CHEMICAL ABSTRACTS,* 1641-17-4
- *CHEMICAL ABSTRACTS,* 1843-05-6
- CTFA Cosmetic Ingredient Directory